# EUROPEAN PATENT APPLICATION

(11) **EP 4 393 439 A1**
(43) Date of publication of application: **03.07.2024**
(21) Application number: 23196836.3
(22) Date of filing: 12.09.2023
(51) Int. Cl.: A61B 34/00, H01F 3/00, H01F 7/06, A61B 6/00, A61B 90/00

(54) **MAGNETIC ACTUATION SYSTEM HAVING CURVED MAGNETIC CORE**

(30) Priority: 26.12.2022 KR 20220184433
(71) Applicant: Daegu Gyeongbuk Institute Of Science and Technology, Daegu 42988 (KR)
(72) Inventor: Choi, Hong Soo, 42951 Daegu (KR); Lee, Hak Joon, 43014 Daegu (KR); Kim, Jin Young, 43016 Daegu (KR); Abbasi, Sarmad Ahmad, 42988 Daegu (KR); Ahmed, Awais, 42988 Daegu (KR); Chowdhury, A M Masum Bulbul, 43014 Daegu (KR); Nader, Latifi Gharamaleki, 42988 Daegu (KR)
(74) Representative: Reiser & Partner Patentanwälte mbB

(57) **Abstract**

The present disclosure provides a magnetic actuation system including a curved magnetic core forming a magnetic field or magnetic force through current supplied thereto to control a therapeutic magnetic actuation robot, the magnetic actuation system including an electromagnet array including a plurality of electromagnets aligned in an arrangement space, wherein each of the plurality of electromagnets includes an electromagnet body linearly extending while including a magnetic core and a coil wound around the magnetic core, and a magnetic core front end portion extending from the magnetic core to protrude from the electromagnet body, and the magnetic core front end portions of the plurality of electromagnets are oriented to be bent toward a target position.

## Description

### CROSS-REFERENCE TO RELATED APPLICATION

This application claims priority to and the benefit of Korean Patent Application No. 10-2022-0184433, filed on December 26, 2022, the disclosure of which is incorporated herein by reference in its entirety.

### BACKGROUND

### 1. Field of the Invention

The present disclosure relates to a magnetic actuation system for controlling a magnetic micro robot or a magnetic actuation robot for treatment such as a magnetic actuation-type catheter, guide wire, or the like using a magnetic field, and more specifically, to a magnetic actuation system having a curved magnetic core.

### 2. Discussion of Related Art

A micro robot or catheter has been mainly developed as a means capable of effectively performing a medical procedure, which is difficult for a human to directly perform, such as being inserted into the human body to diagnose diseases, deliver drugs, or the like.

Specifically, a magnetic micro robot is a technology which is minimally invasive and can precisely deliver a targeted drug, and is expected to dramatically overcome side effects of existing surgical therapies and drug treatments. A method of precisely delivering a cell therapy product such as a drug or a stem cell combined with magnetic nanoparticles to a target position such as a patient's affected area can be used to develop completely new treatment techniques capable of improving an existing treatment method or treating diseases having limitations with conventional treatment methods.

A magnetic actuation system is used to accurately move a magnetic micro robot or a magnetic actuation robot for treatment such as a magnetic actuation-type catheter, guide wire, or the like to a target position such as a patient's affected area. The magnetic actuation system precisely controls the magnetic actuation robot using a magnetic field formed by an electromagnet or a permanent magnet so that the magnetic actuation robot wirelessly moves to a target position. This magnetic actuation system is an electromagnet array in which a plurality of electromagnets are arranged in a predetermined arrangement, and various types of magnetic fields such as a uniform magnetic field, a magnetic field gradient, a rotating magnetic field, an alternating magnetic field, and the like can be formed.

Meanwhile, a treatment technique using the magnetic actuation robot is used with a medical imaging device capable of checking a position of the magnetic actuation robot in the body. A C-arm X-ray device is the most frequently used imaging device for real-time imaging.

However, a conventional magnetic actuation system has a problem of being difficult to install due to spatial interference with the medical imaging device, for example, a C-arm X-ray system.

Each electromagnet of the electromagnet array of the magnetic actuation system should be aligned to be generally arranged on a lower surface of a bed on which a patient lies or an animal test target is placed so that the magnetic field is directed toward a target position such as an affected area. Accordingly, a plurality of electromagnets are spaced apart at a predetermined distance from an arrangement space on the lower surface of the bed to be radially arranged. However, this radial arrangement requires a large arrangement space. Accordingly, there is a disadvantage in that the electromagnets are difficult to install in a limited empty space of a medical imaging device having a complicated structure.

In the magnetic actuation system, the strength of an output magnetic field and magnetic force increase as the number of electromagnets constituting the electromagnet array increases, and the number of electromagnets, which can be radially arranged, is limited due to a spatial limitation by the medical imaging device. That is, there is a limitation of increasing the output magnetic field and magnetic force of the magnetic actuation system.

In addition, in the case of a medical imaging device such as the C-arm X-ray device in which an operating unit including an X-ray reception unit and an X-ray transmission unit moves and captures images, since the electromagnet array should be arranged to prevent the occurrence of interference between the electromagnet array and the operating unit, so that it is not easy to arrange the electromagnet array.

Accordingly, there is a demand for the development of a magnetic actuation system having an electromagnet array that can be arranged while occupying a small area within the arrangement space.
(Patent Document 1) International Patent Publication No. 2018-030610 (published on February 15, 2018)

### SUMMARY OF THE INVENTION

The present disclosure is devised to solve the above-described problems, and the present disclosure is directed to providing a magnetic actuation system having a curved magnetic core including an electromagnet array including a plurality of electromagnets extending in a curved manner and each having a magnetic core front end portion directed toward a target position.

Also, the present disclosure is directed to providing a magnetic actuation system having a curved magnetic core capable of reducing the area occupied by an electromagnet array in an arrangement space where an electromagnet array is arranged.

The present disclosure provides a magnetic actuation system including a curved magnetic core forming a magnetic field or magnetic force through current supplied thereto to control a therapeutic magnetic actuation robot, and the magnetic actuation system includes an electromagnet array including a plurality of electromagnets aligned in an arrangement space. Each of the plurality of electromagnets includes an electromagnet body linearly extending while including a magnetic core and a coil wound around the magnetic core, and a magnetic core front end portion extending from the magnetic core to protrude from the electromagnet body, and the magnetic core front end portions of the plurality of electromagnets are oriented to be bent toward a target position for movement of the therapeutic magnetic actuation robot.

According to an embodiment of the present disclosure, the plurality of electromagnets of the electromagnet array may be arranged in two rows facing each other with a separation space formed therebetween, and the magnetic core front end portions may protrude toward the separation space.

According to the embodiment of the present disclosure, the electromagnet array may be a planar electromagnet array in which the plurality of electromagnets are arranged on a plane.

According to the embodiment of the present disclosure, the electromagnet array may have a free space between the plurality of electromagnets in each row to form a crossing separation space crossing the separation space. The separation space and/or crossing separation space may form a movement path of an operating unit of an imaging device such as a medical imaging device.

According to the embodiment of the present disclosure, the electromagnet array may include a magnetic yoke made of a magnetic material and configured to connect at least some of rear ends of the magnetic cores facing the magnetic core front end portions of the electromagnets.

According to the embodiment of the present disclosure, the magnetic yoke may have a closed frame shape formed to surround the electromagnet array, and configured to connect all of the plurality of electromagnets.

According to the embodiment of the present disclosure, the magnetic yoke may have a plurality of magnetic yoke portions configured to respectively connect the plurality of electromagnets in the two rows of the electromagnet array, and the magnetic yoke portions may be separated from each other.

According to the embodiment of the present disclosure, the arrangement space may be a lower surface of a bed on which a photographing target of the medical imaging device is placed, the medical imaging device may be a C-arm imaging device having an arc including a C-shaped arc body, a reception unit provided at one end of the arc body, and a transmission unit provided at the other end of the arc body, and the arrangement space may be located between the reception unit and the transmission unit.

### BRIEF DESCRIPTION OF THE DRAWINGS

The above and other objects, features, and advantages of the present disclosure will become more apparent to those of ordinary skill in the art by describing exemplary embodiments thereof in detail with reference to the accompanying drawings, in which:
FIG. 1 illustrates an implementation example of a magnetic actuation system according to one embodiment of the present disclosure, and is a perspective view of a magnetic actuation system integrated with a C-arm imaging device;
FIG. 2 is a view exemplarily illustrating the movement of an arc in the C-arm imaging device in which the magnetic actuation system is implemented;
FIG. 3 is a view illustrating an example of an electromagnet array of the magnetic actuation system according to the present disclosure;
FIG. 4 is a view illustrating an example in which a crossing separation space is formed in the electromagnet array according to the present disclosure;
FIG. 5 is a view illustrating a form in which the electromagnet array according to the present disclosure includes a magnetic yoke of an example;
FIG. 6 is a view illustrating a form in which the electromagnet array according to the present disclosure includes a magnetic yoke of another example;
FIG. 7 is a view illustrating a magnetic field simulation result of electromagnets for comparing and showing a magnetic flux density (a) formed by an electromagnet including a linear magnetic core and a magnetic flux density (b) formed by an electromagnet including a curved magnetic core according to the present disclosure;
FIG. 8 is a view illustrating a magnetic field simulation result by an electromagnet array in which six electromagnets including curved magnetic cores are arranged in a planar array of two rows in the magnetic actuation system according to the present disclosure; and
FIG. 9 is a view of a simulation result showing an output magnetic field in an example in which the electromagnet array of the magnetic actuation system according to the present disclosure includes the magnetic yoke.

### DETAILED DESCRIPTION OF EXEMPLARY EMBODIMENTS

Since the present disclosure may be variously changed and have various embodiments, specific embodiments will be illustrated in the drawings and described in detail in the detailed description. However, this is not intended to limit the present disclosure to specific embodiments, and it should be understood that the specific embodiments include all changes, equivalents, and substitutes included in the spirit and scope of the present disclosure. In the description of the present disclosure, when it is determined that detailed descriptions of related known technologies may obscure the gist of the present disclosure, the detailed description thereof will be omitted.

Terms such as first, second, and the like can be used to describe various components, but the components should not be limited by the terms. The terms are only used to distinguish one component from another component.

Terms used in the present application are only used to describe specific embodiments, and are not intended to limit the present disclosure. A singular form includes a plural form unless otherwise defined.

Hereinafter, embodiments of the present disclosure will be described in detail with reference to the accompanying drawings.

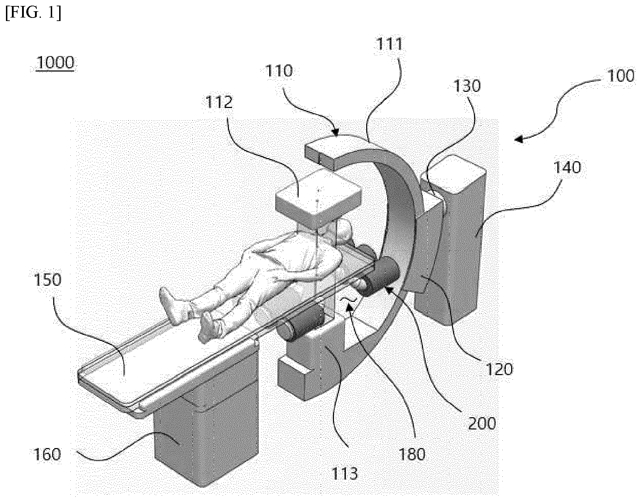

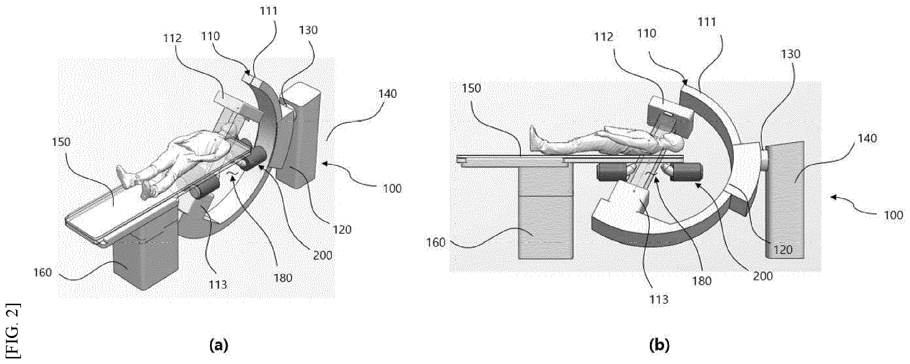

FIG. 1 illustrates an implementation example of a magnetic actuation system according to one embodiment of the present disclosure, and is a perspective view of a magnetic actuation system integrated with a C-arm imaging device, and FIG. 2 illustrates the movement of an arc in the C-arm imaging device in which the magnetic actuation system is implemented, wherein FIG. 2A illustrates the sliding movement of the arc and FIG. 2B illustrates the rotational movement of the arc.

Referring to FIG. 1, a magnetic actuation system 1000 according to one embodiment of the present disclosure includes an electromagnet array 200 and a C-arm imaging device 100.

The C-arm imaging device 100 includes an arc 110, a tray 120, an actuation unit 130, a body part 140, and a bed 150.

The arc 110 is formed in a C shape and is slidably mounted in a circumferential direction with respect to the tray 120. The arc 110 includes a C-shaped arc body 111, a reception unit 112 provided at one end of the arc body 111, and a transmission unit 113 which is connected to the other end of the arc body 111 and generates X-rays.

The transmission unit 113 includes an X-ray generation tool for generating X-rays, and the X-rays may be transmitted to the reception unit 112 after passing through a photographing target such as a patient provided between the transmission unit 113 and the reception unit 112.

The reception unit 112 is configured to convert X-ray photons that have passed through the target into an image that can be recognized by the human eye.

The tray 120 is supported so that the arc 110 may slide in the circumferential direction, and is connected to the actuation unit 130. The actuation unit 130 is supported by the body part 140 and supports the tray 120 so that the tray 120 is rotatably movable. Accordingly, sliding movement of the arc 110 on the tray 120 as shown in FIG. 2A and rotation of the tray 120 relative to the actuation unit 130 as shown in FIG. 2B are possible, and accordingly, translational and rotational movement of the arc 110 in three dimensions is possible.

The body part 140 is connected to an operating unit (not shown) including a user interface, and the operating unit is configured to be capable of transmitting signals to the transmission unit or a control unit through a touch panel or the like, and to check information sensed by the reception unit as a form of image information through a display.

The bed 150 is supported by a support 160 and extends into a space between the transmission unit 113 and the reception unit 112, and a target such as a patient or the like lies or is placed thereon. The space above the bed 150 between the transmission unit 113 and the reception unit 112 becomes a procedural space.

The magnetic actuation system 1000 includes an electromagnet array 200 including a plurality of electromagnets applied to the C-arm imaging device 100, and the electromagnet array 200 is arranged in an arrangement space 180 formed under the bed 150 between the transmission unit 113 and the reception unit 112.

The magnetic actuation system 1000 according to one embodiment of the present disclosure shown in the drawings is applied to a C-arm imaging device, but the magnetic actuation system 1000 may form one system with another medical imaging device or medical device, or may be present as a separate system. The present disclosure is not limited by these embodiments. The magnetic actuation system 1000 may include various components such as a current supply device, a cooling device, a support device, and the like, which are not shown.

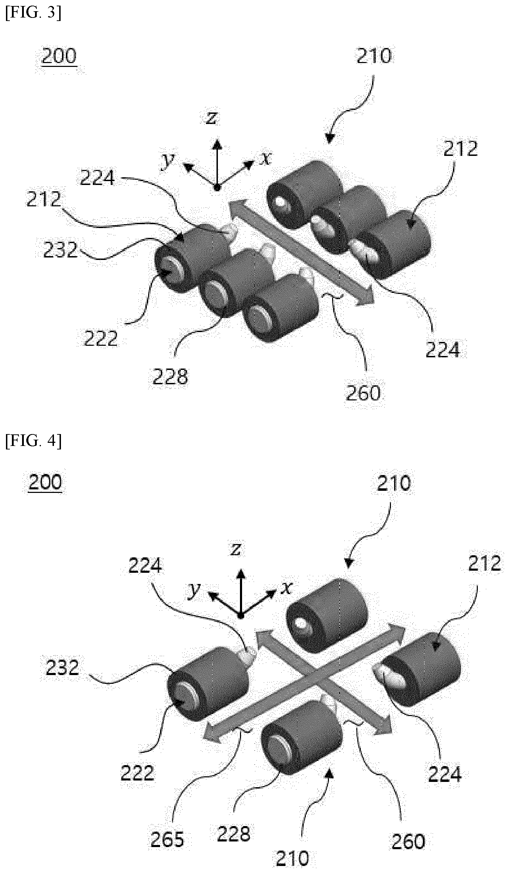

FIG. 3 is a view illustrating an example of the electromagnet array 200 of the magnetic actuation system 1000 according to the present disclosure.

A magnetic material receives a magnetic torque aligned in a direction of the magnetic field, and receives a magnetic force to move to a position where the strength of the magnetic field is strong. Therefore, by controlling the distribution of the magnetic field around the magnetic material, torque and force may be wirelessly applied to the magnetic material. Accordingly, the magnetic actuation system according to one embodiment of the present disclosure may control an end portion of a micro robot or catheter in the three dimension by controlling the magnetic field distribution around the end portion of the micro robot, catheter, or guide wire including a magnetic material to wirelessly apply torque and power to the magnetic material. In the present disclosure, a magnetic micro robot, magnetic actuation-type catheter, guide wire, or the like including magnetic micro/nano-scaled materials is called a magnetic actuation robot. Since the magnetic actuation-type catheter or guide wire includes a magnetic material in a front end portion, a movement path of the front end portion is controlled by the magnetic actuation system.

According to the embodiment of the present disclosure, the magnetic actuation robot may be a magnetic micro robot including magnetic micro/nanoparticles, and the magnetic micro robot may have a form in which particles capable of forming images for diagnosis, therapeutic cells, therapeutic drugs, or the like are loaded in the magnetic micro/nanoparticles or the magnetic micro/nanoparticles are formed to enable thermal therapy or an electrical stimulation therapy.

The electromagnet array 200 according to the present disclosure includes a plurality of electromagnets 210. The electromagnet array 200 is configured to generate a desired three-dimensional magnetic field and magnetic force by controlling the current applied to each of the electromagnets 210 of the electromagnet array 200.

The electromagnet 210 includes an electromagnet body 212 including a magnetic core 222 and a coil 232 wound around the magnetic core 222 and extending linearly, and a magnetic core front end portion 224 protruding from the electromagnet body 212 while being integrated with the magnetic core 222. The magnetic core front end portion 224 protrudes into a separation space 260 which is a space between two rows of the electromagnet array 200.

The magnetic core front end portion 224 is integrally formed with the magnetic core 222, and is a portion of the magnetic core 222. However, in the present specification, for the purpose of description, in the magnetic core, a portion protruding from the electromagnet body 212, which is a portion on which the coil 232 is uniformly wound, and extends linearly, is separately called the magnetic core front end portion 224.

The magnetic core front end portion 224 has a shape extending in a curved manner so that an extension path thereof does not coincide with the electromagnet body 212 which extends linearly. The magnetic core front end portion 224 extends in a curved manner from the linear electromagnet body 212, and thus may be distinguished from the electromagnet body 212.

An end portion of the magnetic core 222 facing the magnetic core front end portion 224 is called a rear end 228.

According to the present disclosure, as will be described below, the magnetic core 222 has a magnetic core front end portion 224 at least bent in a curved shape, and thus may be called a curved magnetic core as a whole.

According to the embodiment of the present disclosure, the magnetic core front end portion 224 of each of the plurality of electromagnets 210 of the electromagnet array 200 is oriented to face a set target position. This target position is located in a z-axis direction with respect to the electromagnet array 200 arranged on an x-y plane.

Referring to FIG. 3, it can be seen that the coil front end portions 224 of the plurality of electromagnets 210 are respectively bent in different directions toward the target position located in a +z-axis direction according to arrangement positions. Like the above, since the coil front end portions 224 of the plurality of electromagnets 210 are arranged to face the target position, the magnetic actuation robot placed at the target position may receive a strong magnetic field and magnetic force for generating a desired movement.

According to the present disclosure, in the electromagnet array 200, the electromagnets 210 are arranged on the same plane on the x-y plane. Accordingly, the electromagnet bodies 212 may be arranged side by side

Since the plurality of electromagnets 210 are arranged on a plane, and the magnetic core front end portions 224 are arranged to be bent and oriented toward the target position, it is possible to reduce an area occupied by the electromagnet array 200 compared to a known arrangement structure in which the plurality of electromagnets 210 are radially arranged toward the target position.

The plurality of electromagnets 210 of the electromagnet array 200 may form an arrangement of two rows facing each other with the separation space 260 therebetween. The electromagnet array according to the present disclosure includes at least four electromagnets 210 whose positions are fixed on the same plane. Referring to FIG. 3, the electromagnet array 200 is composed of six electromagnets 210, three electromagnets 210 along each row are arranged in an arrangement so that the electromagnet bodies 212 are side by side, and the electromagnets 210 face the electromagnets 210 in the opposite row with the separation space 260 therebetween.

This arrangement allows the electromagnets 210 in the rows to be arranged adjacent to each other, and thus allows the occupied area to be reduced, and the separation space 260 provides movement paths for an end portion of the arc 110 of the C-arm imaging device. The movement paths of the arc 110 are indicated by arrows in FIG. 4. Like the above, since the present disclosure makes it easy to secure the movement paths of the arc 110 while reducing the area occupied by the electromagnet array 200 compared to the radial arrangement, the present disclosure allows the electromagnets 210 to be arranged in a concentrated manner in a compact arrangement space while preventing interference with other medical devices which are installed together.

FIG. 4 illustrates an example in which a crossing separation space is formed in the electromagnet array according to the present disclosure.

The electromagnet array 200 according to the present disclosure has a free space, that is, a crossing separation space 265 formed by the plurality of electromagnets 210 in each row and not having the electromagnets 210, so that the crossing separation space 265 vertically crossing the separation space 260 is formed.

FIG. 4 illustrates an electromagnet array 200 in which two electromagnets 210 are arranged along each row, and illustrates that the crossing separation space 265 where the arc 110 of the C-arm imaging device 100 may move is formed between the two electromagnets 210 forming each row. The arrows in FIG. 4 indicate the movement paths of the arc 110.

Even though the electromagnet array 200 according to the present disclosure is arranged in the arrangement space 180 located on a lower surface of the bed 150, the electromagnet array 200 may include the separation space 260 and the crossing separation space 265 forming the movement paths of the arc 110 in the electromagnet array 200.

Accordingly, both rotation during a sliding movement of the arc of the C-arm imaging device on the tray shown in FIG. 2A and three-dimensional rotation during rotation of the actuation unit shown in FIG. 2B may be performed without interference.

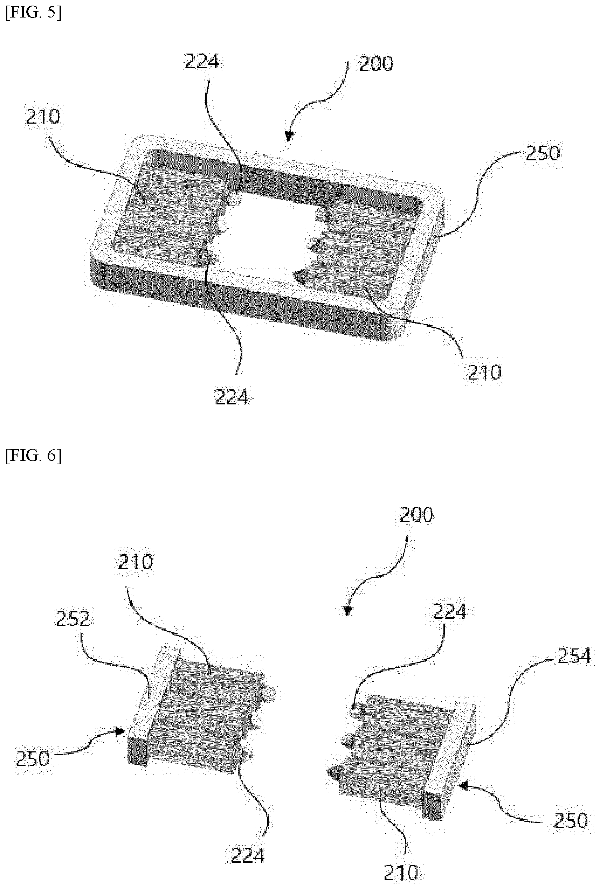

FIGS. 5 and 6 illustrate embodiments in which the electromagnet array according to the present disclosure includes a magnetic yoke.

As shown in FIGS. 5 and 6, the electromagnet array 200 according to the present disclosure may further include a magnetic yoke 250 formed of a magnetic material which at least partially connect the rear ends 228 of the magnetic cores 222.

The magnetic material constituting the magnetic yoke 250 may be the same material constituting the magnetic core 222 of the electromagnet, but the present disclosure is not limited thereto, and various magnetic materials usable as the magnetic core 222 may be used.

Referring to FIG. 5, the magnetic yoke 250 may be formed in a closed frame form which connects all of the plurality of electromagnets 210 of the electromagnet array 200. Referring to FIG. 5, the magnetic yoke 250 has a quadrangular frame form, and connects all of the rear ends 228 of the plurality of electromagnets 210.

This closed frame form increases the output magnetic field and magnetic force of the electromagnet array by forming a closed magnetic circuit.

Referring to FIG. 6, the magnetic yoke 250 of the electromagnet array 200 according to the present disclosure includes a plurality of magnetic yoke portions 252 and 254 which at least partially connect the plurality of electromagnets in each row. The magnetic yoke portion 252 at one side has a rod-shaped arrangement which connects the rear ends 228 of the electromagnets 210 in one row in the two-row arrangement of the plurality of electromagnets 210, and the magnetic yoke portion 254 at the other side connects the rear ends 228 of the electromagnets 210 in the other row in the two-row arrangement of the plurality of electromagnets 210.

The magnetic yoke 250 in the form of including the plurality of magnetic yoke portions 252 and 254 separated from each other has a weak increase in the output magnetic field and magnetic force compared to the closed frame form, but increases the magnetic field compared to a form without the magnetic yoke 250.

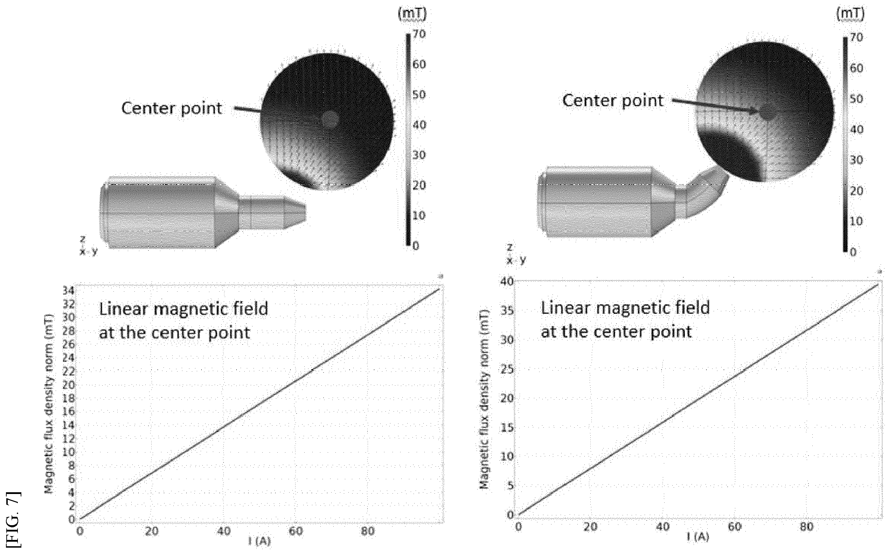

FIG. 7 is a view illustrating a magnetic field simulation result of the electromagnets in which a magnetic flux density (a) formed in a linear magnetic core and a magnetic flux density (b) formed in a curved magnetic core are compared.

The electromagnets are arranged in a planar arrangement in the arrangement space, and a patient's affected area forming the target position, that is, a work space for controlling the magnetic actuation robot is located above the arrangement space.

As shown in FIG. 7, it can be seen that the magnetic flux density (b)formed by the magnetic core in which the magnetic core extends in a curved shape, that is, the magnetic core having the magnetic core front end portion bent toward a target position, is further improved compared to the magnetic flux density (a) generated by the electromagnet in which the magnetic core linearly extends. That is, a stronger magnetic field/magnetic force may be formed in the work space by the electromagnet including the curved magnetic core.

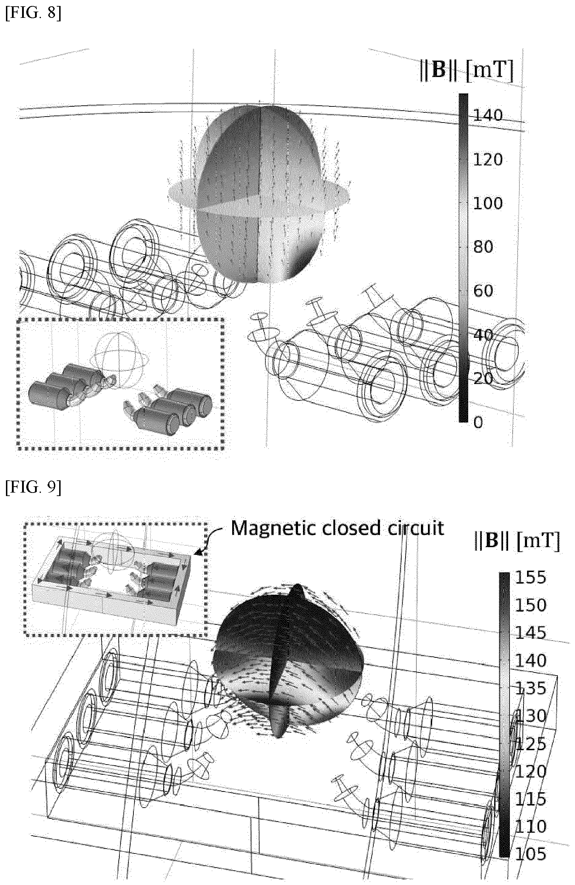

FIG. 8 is a view illustrating a simulation result by the electromagnet array in which the electromagnets including the curved magnetic cores are arranged in a planar array of two rows in the magnetic actuation system according to the present disclosure. FIG. 8 shows an output in the +z-axis direction as a magnetic field simulation result of an arrangement of six electromagnets in the electromagnet array according to the present disclosure.

The desired three-dimensional magnetic field and magnetic force may be generated by controlling a current applied to each electromagnet through the electromagnet array according to the present disclosure in which the electromagnets are arranged on the same plane, and the strength of the output magnetic field/ magnetic force increases as the number of electromagnets forming the electromagnet array increases.

FIG. 9 illustrates a simulation result confirming that the output magnetic field increase when the electromagnet array of the magnetic actuation system according to the present disclosure includes a magnetic yoke, and illustrates an output in an x direction.

Referring to FIG. 9, the output magnetic field and magnetic force may be increased when the electromagnet array further include a magnetic yoke, and the increase in the output magnetic field and magnetic force is excellent in a closed frame form.

A magnetic actuation system according to the present disclosure includes an electromagnet array in which electromagnets including coil front end portions bent in a curved shape and oriented to a target position and curved magnetic cores are arranged, and thus has an advantage in that the electromagnets including the curved magnetic cores can be arranged to be freely directed toward a target position from arrangement positions. Accordingly, the degree of freedom of arrangement of the electromagnets in the electromagnet array increases.

Further, according to the present disclosure, it is possible to reduce the area occupied by the electromagnet array in an arrangement space by allowing a plurality of electromagnets of the electromagnet array to be arranged on a plane. This reduction in occupied area makes it possible to arrange the electromagnet array in limited spaces of the various medical devices, thereby enabling the implementation of a magnetic actuation system applied to various medical devices.

According to the present disclosure, the planar arrangement of the plurality of electromagnets of the electromagnet array allows, for example, arrangements for easily securing a movement path of an arc of a C-arm imaging device.

According to the present disclosure, the electromagnet array includes a magnetic yoke, which makes it possible to increase an output magnetic field and magnetic force.

The present disclosure should not be interpreted as technical spirit limited to the above-described embodiments. The scope of application is diverse, and various modifications may be performed by those skilled in the art without departing from the gist of the present disclosure claimed in the claims. Accordingly, such improvements and changes fall within the scope of the present disclosure as long as they are obvious to those skilled in the art.

## Claims

1. A magnetic actuation system including a curved magnetic core forming a magnetic field or magnetic force through current supplied thereto to control a therapeutic magnetic actuation robot, the magnetic actuation system comprising an electromagnet array including a plurality of electromagnets aligned in an arrangement space,
wherein each of the plurality of electromagnets includes an electromagnet body linearly extending while including a magnetic core and a coil wound around the magnetic core, and a magnetic core front end portion extending from the magnetic core to protrude from the electromagnet body, and
the magnetic core front end portions of the plurality of electromagnets are oriented to be bent toward a target position for movement of the therapeutic magnetic actuation robot.

2. The magnetic actuation system of claim 1, wherein the plurality of electromagnets of the electromagnet array are arranged in two rows facing each other with a separation space formed therebetween, and
the magnetic core front end portions protrude toward the separation space.

3. The magnetic actuation system of claim 2, wherein the plurality of electromagnets are arranged on a plane in the electromagnet array.

4. The magnetic actuation system of claim 3, wherein the electromagnet array has a free space between the plurality of electromagnets in each row to form a crossing separation space crossing the separation space.

5. The magnetic actuation system of claim 3 or 4, wherein the electromagnet array includes a magnetic yoke made of a magnetic material and configured to connect at least some of rear ends of the magnetic cores facing the magnetic core front end portions of the electromagnets.

6. The magnetic actuation system of claim 5, wherein the magnetic yoke has a closed frame shape formed to surround the electromagnet array, and configured to connect all of the plurality of electromagnets.

7. The magnetic actuation system of claim 5, wherein the magnetic yoke has a plurality of magnetic yoke portions configured to respectively connect the plurality of electromagnets in the two rows of the electromagnet array, and
the magnetic yoke portions are separated from each other.

8. The magnetic actuation system of claim 1 or 3, wherein the arrangement space is a lower surface of a bed on which a photographing target of a medical imaging device is placed.

9. The magnetic actuation system of claim 8, wherein the medical imaging device is a C-arm imaging device having an arc including a C-shaped arc body, a reception unit provided at one end of the arc body, and a transmission unit provided at the other end of the arc body, and
the arrangement space is located between the reception unit and the transmission unit.
